(19) Europäisches Patentamt
European Patent Office
Office européen des brevets

(11) Publication number: 0 130 554
B1

(12) EUROPEAN PATENT SPECIFICATION

(45) Date of publication of patent specification: 24.02.88

(51) Int. Cl.$^4$: C 07 D 217/16, C 12 P 17/12, A 61 K 31/47 // C12R1/465

(21) Application number: 84107409.9

(22) Date of filing: 28.06.84

# (54) Novel, physiologically active substance, process for production thereof and pharmaceutical composition containing the same.

(30) Priority: 30.06.83 JP 119415/83

(43) Date of publication of application: 09.01.85 Bulletin 85/02

(45) Publication of the grant of the patent: 24.02.88 Bulletin 88/08

(84) Designated Contracting States: DE FR GB IT

(56) References cited: GB-A-1 374 299

(73) Proprietor: KYOWA HAKKO KOGYO CO., LTD.
6-1, Ohte-Machi 1-chome
Chiyoda-ku Tokyo-to (JP)

(72) Inventor: Kase, Hiroshi
3-35-18, Maehara-cho
Koganei-shi Tokyo (JP)
Inventor: Fujita, Hironori
3-3-8-503, Azamino Midori-ku
Yokohama-shi Kanagawa-ken (JP)
Inventor: Iida, Takao
3-2-22, Katsuma
Hofu-shi Yamaguchi-ken (JP)
Inventor: Shirahata, Kunikatsu
4-11-5, Iwatominami
Komae-shi Tokyo (JP)
Inventor: Hirayama, Noriaki
638-2, Tatsumodai
Zama-shi Kanagawa-ken (JP)
Inventor: Nakayama, Kiyoshi
5-16-9, Minamidai
Sagamihara-shi Kanagawa-ken (JP)
Inventor: Kubo, Kazuhiro
625-11, Kashiwakubo Shuzenji-machi
Tagata-gun Shizuoka-ken (JP)
Inventor: Nakamura, Joji
1188, Shimotogari Nagaizumi-cho
Sunto-gun Shizuoka-ken (JP)

EP 0 130 554 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Casalonga, Axel et al**
**BUREAU D.A. CASALONGA OFFICE JOSSE &**
**PETIT Morassistrasse 8**
**D-8000 München 5 (DE)**

**Description**

Background of the Invention

This invention relates to a novel, physiologically active substance and a process for producing the same.

New physiologically active substances useful as medicaments are always in demand.

As a result of studies on products from micro-organisms available in the natural field, it has been found that a physiologically active substance having an activity to block a sympathetic nerve receptor is produced in a culture liquor of a microorganism belonging to the genus *Streptomyces,* and further it has been found by investigation of its physico-chemical properties that the substance isolated and purified from the culture liquor is a novel, physiologically active substance, having a tetrahydroisoquinoline ring. The substance has been named MY336-a.

Heretofore, there have been known the compounds represented by the following formulae, having an activity to block a sympathetic nerve receptor and a tetrahydroisoquinoline ring [Y. Nimitkitpaisan and P. Skolnick, Life Science, *23,* 375—382 (1978)].

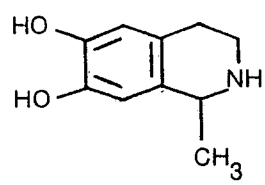

Salsolinol

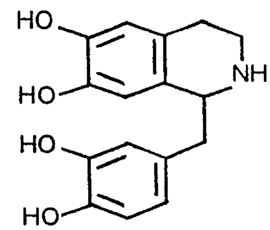

Tetrahydropapaveroline

These compounds have both antagonistic and agonistic activities. MY336-a has a quite different structure from these compounds, and has no agonistic activity on adrenergic receptor, while it has an antagonistic activity.

The properties of MY336-a, a process for producing the substance, its pharmacological action and a pharmaceutical composition containing the substance are described hereinafter.

Brief Description of Drawings

Fig. 1 is an ultraviolet absorption spectrum of MY336-a; where curve 1 illustrates the results of measurement at the neutral point, curve 2 those at an acidic side and curve 3 those at an alkaline side.

Fig. 2 is an infrared absorption spectrum of MY336-a measured by KBr tablet method.

Detailed Description of the Invention

The physiologically active substance MY336-a according to the present invention has the following physico-chemical properties:

1 State: colorless, needle-like crystal

2 Melting point: 177—178°C

3 Elemental analysis (%):

| | Found | Calculated (as $C_{13}H_{19}NO_4$) |
|---|---|---|
| C | 61.60 | 61.64 |
| H | 7.58 | 7.56 |
| N | 5.40 | 5.53 |

4 Specific rotation: $[\alpha]_D = -127°$ (c=0.5, methanol).

5 Solubility: readily soluble in acidic water, soluble in water and methanol, and sparingly soluble in acetone, hexane and ether.

6 Ultraviolet absorption spectrum: As is illustrated in Fig. 1, where curve 1 illustrates the results of measurement at the neutral point, curve 2 those at an acidic side, and curve 3 those at an alkaline side.

7 Infrared absorption spectrum (KBr tablet method): as is illustrated in Fig. 2.

8 PMR spectrum: PMR spectrum ($CD_3OD$) of the substance shows the following δ values (unit: ppm): 2.20 (3H, s), 2.40—2.55 (2H, m), 2.64—2.90 (1H, m), 3.48—3.78 (2H, m), 3.69 (3H, s), 3.78 (1H, dd, J=6.1, 10.5), 4.12 (1H, dd, J=3.4, 10.5), 4.28 (1H, m), 6.43 (1H, s).

9 CMR spectrum: CMR spectrum ($CD_3OD$) of the substance shows the following δ value: 15.7, 33.6, 55.6, 56.6, 60.7, 65.7, 66.5, 121.5, 122.6, 130.1, 133.9, 145.6, 148.2.

It can be presumed from the foregoing physico-chemical properties that MY336-a is a novel substance having the following chemical structure of formula (I):

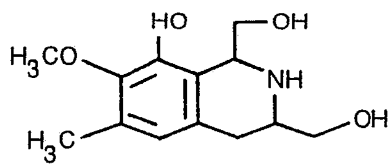

(I)

The $R_f$ values of MY336-a on thin layer chromatography by various developing solvents are given in Table 1.

Table 1

$R_f$ values on silica gel thin layer chromatography [kieselgel 60, HPTLC Fertigplatten, 10 cm × 10 cm, made by E. Merck]

| Developer* | Developing time | $R_f$ value |
|---|---|---|
| I | 25 minutes | 0.02 |
| II | 1 hour 20 minutes | 0.29 |
| III | 30 minutes | 0.43 |

*Developer I: chloroform:methanol (9:1 by volume)
Developer II: n-propanol:ethyl acetate:water (1:1:1 by volume)
Developer III: acetic acid:chloroform:methanol (1:7:2 by volume)

A process for producing MY336-a is described hereinafter.

MY336-a can be obtained by culturing an MY336-a producing strain belonging to the genus *Streptomyces* in a nutrient medium, thereby forming and accumulating MY336-a in the culture liquor, and recovering the substance from the culture liquor.

The specific examples of MY336-a producing strains belonging to the genus *Streptomyces* is *Streptomyces gabonae* ATCC 15282, which is deposited with and available to the public from the American Type Culture Collection.

Culturing for producing MY336-a is carried out in the following manner.

Ordinary procedures for culturing the genus *Actinomyces* may be employed for culturing the present MY336-a producing strains.

Either a synthetic medium or a natural medium may be used as long as it contains suitable carbon sources and nitrogen sources.

As a carbon source, glucose, starch, dextrin, mannose, fructose, sucrose or molasses can be used alone or in combination. Furthermore, hydrocarbons, alcohols or organic acids can also be used, depending upon the assimilability of microorganisms.

As a nitrogen source, inorganic and organic nitrogen-containing compounds such as ammonium chloride, ammonium sulfate, urea, ammonium nitrate or sodium nitrate and nitrogen-containing natural products such as peptone, meat extract, yeast extract, dry yeast, corn steep liquor, soybean meal, casamino acid, soluble vegetable protein or cotton seed residues can be used alone or in combination. If necessary, inorganic salts such as sodium chloride, potassium chloride, calcium carbonate or phosphates can be appropriately used, and organic and inorganic substances capable of promoting growth of the microorganism used for production of MY336-a can also be appropriately used.

A liquid culturing method, particularly a submerged stirring culture method is most suitable for culturing strains of the present invention. Culturing is desirably carried out at a temperature of 25—40°C and at a pH of around neutrality. MY336-a is formed and accumulated in the culture liquor usually after 1—12 days of liquid culturing. When the accumulation in the culture liquor reaches a maximum, culturing is discontinued and the desired substance is isolated and purified from the filtrate of the culture liquor obtained by filtering off the cells.

Ordinary procedures for isolating metabolic products of microorganisms from a culture liquor filtrate may be used for isolation and purification of MY336-a. That is, absorption and desorption by active carbon, Diaion HP—10 (trademark of Mitsubishi Kasei Kogyo Co., Ltd.), column chromatography by various ion exchange resins, cellulose column chromatography, silica gel column chromatography, CM-Sephadex (trademark of Pharmacia Fine Chemicals Co.) column chromatography, SP-Sephadex column chromatography or Sephadex LH—20 column chromatography can be used in an appropriate combination.

As an example of an isolation and purification procedure, the culture liquor filtrate is adjusted to pH 4 with hydrochloric acid and then passed through a column filled with Diaion HP—10. Then, the column is thoroughly washed and eluted with an aqueous 50% methanol solution. Fractions containing MY336-a are joined together, and methanol is removed therefrom by distillation under reduced pressure. Then, the

residue is passed through a column of Diaion SK—1B ($NH_4^+$). After washing with water, the column is eluted with 2N aqueous ammonia. Fractions containing MY336-a are joined together and concentrated under reduced pressure.

The concentrate is neutralized, and passed through a column of Sephadex C—25. After washing with water, the column is eluted with a concentration gradient of 0—0.05M phosphate buffer (pH 8.0). Fractions containing MY336-a are joined together, concentrated under reduced pressure, and freeze dried, whereby a crude preparation of MY336-a is obtained. The crude preparation is subjected to silica gel chromatography with a solvent of n-butanol:ethanol:chloroform:concentrated aqueous ammonia (4:5:2:2), and then fractions containing MY336-a are joined together and concentrated to dryness under reduced pressure. The residue is dissolved in a small amount of methanol, and the solution is allowed to stand at room temperature to deposit crystals of MY336-a. By recrystallization of the crytals from methanol, pure, colorless, needle-like crystals of MY336-a can be obtained.

MY336-a has an activity to block a sympathetic nerve receptor and can be applied to curing of hypertension, arhythmia, myocardial infraction, angina pectoris or anxiety solitary neurosis.

Thus, the invention includes within its scope a pharmaceutical composition comprising, as an active ingredient, MY336-a in asociation with at least one of pharmaceutical carriers or diluents. MY336-a is administered by parenteral (intramuscular, intraperitoneal, intravenous or subcutaneous injection routes), oral or rectal route of administration and can be formulated in dosage forms appropriate for each route of administration.

Preparations according to this invention for parenteral administration include sterile aqueous or non-aqueous solutions, suspensions or emulsions. Examples of non-aqueous solvents or vehicles are propylene glycol, polyethylene glycols, vegetable oils such as olive oil, and injectable organic esters such as ethyl oleate. Such dosage forms may also contain adjuvants such as preserving, wetting, emulsifying and dispersing agents. They may be sterilized by, for example, filtration through a bacteria-retaining filter, by incorporating sterilizing agents into the compositions, by irradiating the compositions or by heating the compositions. They can also be manufactured in the form of sterile solid compositions which can be dissolved in sterile water or some other sterile injectable medium immediately before use.

Compositions for oral administration may be presented in a form suitable for absorption by the gastrointestinal tract. Tablets and capsules for oral administration may be in the unit dose presentation form, and may contain conventional excipients such as binding agents, for example, syrup, acacia, gelatin, sorbitol, tragacanth or polyvinyl-pyrrolidone; fillers, for example, lactose, sugar, maize-starch, calcium phosphate, sorbitol or glycine; lubricants, for example, magnesium stearate, talc, polyethylene glycol, silica; disintegrants, for example, potato starch or acceptable wetting agent such as sodium lauryl sulfate. The tablets may be coated according to methods well known in the arts. Oral liquid preparations may be in the form of aqueous or oily suspension, solution, emulsion, syrup or may be presented as a dry product, for reconstitution with water or other suitable vehicle before use. Such liquid preparations may contain conventional additive such as suspending agents, for example, sorbitol syrup, methyl cellulose, glucose sugar syrup, gelatin, hydroxy-ethylcellulose, carboxymethylcellulose, aluminum stearate gel, emulsifying agents, for example, lecithin or sorbitan monooleate; non-aqueous vehicles, which may include edible oils, for example, almond oil, coconut oil, propylene glycol or ethyl alcohol; preservatives, for example, methyl or propyl p-hydroxybenzoates or sorbic acid.

Compositions for rectal administration are preferably suppositories which may contain, in addition to the active substance, excipients such as polyethylene glycols, a triglyceride mixture of saturated fatty acids, cocoa butter or a suppository wax.

The dosage of active ingredient in the compositions of this invention may be varied; however, it is necessary that the amount of the active ingredient shall be such that a suitable dosage form is obtained. The slected dosage depends upon the desired therapeutic effect, on the route of administration, and on the duration of the treatment.

Generally, the present pharmaceutical composition is administered to a human patient as the active ingredient in a dosage of 2—10 mg/kg/day for oral administration, or 0.01—0.2 mg/kg/day for parenteral or rectal administration.

The present invention is illustrated in more detail below, referring to an example and test example.

Example 1

As a seed microorganism, *Streptomyces gabonae* ATCC 15282 is used, and as a first seed medium, a medium containing 1 g/dl glucose, 1 g/dl soluble starch, 0.3 g/dl beef extract, 0.5 g/dl yeast extract, 0.5 g/dl Bactotryptone, and 0.2% of calcium carbonate (pH 7.2 before sterilization) is used. One loopful of the seed microorganism is inoculated in 10 ml of the seed medium in a 50 ml-large test tube and cultured with shaking at 30°C for 4 days. Then, 50 ml of the seed culture liquor (corresponding to the volume of 5 test tubes) is inoculated in 500 ml of a second seed medium in a 2 l-Erlenmeyer flask provided with a baffle. The composition of the second seed medium is the same as that of the first seed medium. The second seed culturing is conducted at 30°C for 2 days, and 500 ml of the second seed culture liquor is inoculated in 15 l of a main fermentation medium in a 30 l-stainless steel jar fermenter. The composition of the main fermentation medium is 3 g/dl dextrin, 2 g/dl soybean meal, 0.25 g/dl corn steep liquor, 0.05 g/dl dipolassium hydrogen phosphate, 0.05 g/dl magnesium sulfate heptahydrate, 0.03 g/dl potassium chloride,

and 0.3 g/dl calcium carbonate (pH 7.8 before sterilization). The main fermentation culturing is conducted at 30°C for 42 hours by aeration-stirring (agitation: 350 rpm, aeration: 15 l/min).

The pH of 30 l of the thus obtained fermented liquor (corresponding to 2 units of said 30 l-jar fermenter) is adjusted to 4.0 with concentrated hydrochloric acid, and the fermented liquor is centrifuged in a Sharpless centrifugal separator to separate a supernatant from the cells. The supernatant is passed through a column filled with 2 l of Diaion HP—10. After washing with 10 l of water, the column is eluted with 10 l of an aqueous 50% (V/V) methanol solution, and the eluate is concentrated to 3 l under reduced pressure. The concentrate is adjusted to pH 5, and passed through a column filled with 2 l of Diaion SK—1B ($NH_4^+$). After washing with 6 l of water, the column is eluted with 6 l of 1N aqueous ammonia. The eluates are joined together and concentrated to 2 l under reduced pressure, and the concentrate is adjusted to pH 7.0 with hydrochloric acid, and passed through a column filled with 600 ml of CM Sephadex C—25. After washing with 1.8 l of water, the column is eluted with 1.8 l of 0.01M phosphate buffer (pH 8), and then further with 1.8 l of 0.05M phosphate buffer (pH 8.0).

Fractions eluted by the 0.05M phosphate buffer are joined together, and passed through a column filled with 300 ml of Diaion SK—1B ($NH_4^+$). After washing with 1 l of water, the column is eluted with 900 ml of 1N aqueous ammonia. The eluates are joined together and concentrated to 100 ml under reduced pressure, and then the concentrate is adjusted to pH 7.0 with hydrochloric acid and passed through a column filled with 400 ml of CM Sephadex C—25. After washing with 1.2 l of water, the column is eluted with a concentration gradient between 2 l of water and 2 l of 0.05M phosphate buffer (pH 8.0).

After initial elution of a few microcomponents, MY336-a is eluted. The fractions containing MY336-a are joined together, and passed through a column filled with 100 ml of Diaion SK—1B ($NH_4^+$). After washing with 300 ml of water, the column is eluted with 1N aqueous ammonia, and the elutate is concentrated to dryness under reduced pressure.

The residue is thoroughly mixed with silica gel, and the mixture is placed on the top end of silica gel column filled using a solvent of n-butanol:ethanol:chloroform:concentrated aqueous ammonia (4:5:2:2). Then, the column is eluted with the same solvent. Fractions containing MY336-a are joined together, and concentrated under reduced pressure to remove the solvent and further to dryness. The residue is dissolved in a small amount of methanol and the solution is left standing at 10°C to deposit crystals. By recrystalllizing the crystals from methanol, 50 mg of colorless, needle-like crystals of pure MY336-a can be obtained. MY336-a in the purification procedure can be monitored by iodine colorization after development in silica gel thin layer chromatography.

Test Example 1

Method:

Atria were rapidly isolated from guinea pigs (body weight: 500—700G) and mounted in an organ bath. The bath was filled with 30 ml oxygenated (95% $O_2$, 5%. $CO_2$) Krebs-Henseleit's solution at 31±1°C. Atria were allowed to beat spontaneously and the contractions were isometrically recorded using force-displacement transducers. Resting force was adjusted to 0.5 g. Then, atria rate was measured. Then, a solution of isoproterenol hydrochloride in a very small amount of water was added thereto to make the isoproterenol hydrochloride concentration to be $10^{-9}$ g/ml, and atrial rate was measured.

The solution was discarded and the bath was filled with Krebs-Henseleit's solution, and allowed to stand until the atrial rate was brought back.

Then, a solution of MY336-a in a very small amount of 50% ethanol was added thereto to make the MY336-a concentration to be 30 μg/ml, and 30 minutes thereafter, a solution of isoproterenol hydrochloride in a very small amount of water was added thereto to make the isoproterenol hydrochloride concentration to be $10^{-9}$ g/ml, and atrial rate was measured.

Inhibitory activity was assessed by comparing positive chronotropic effects due to isoproterenol hydrochloride, which is exhibited by increase of atrial rates, before and after addition of MY336-a.

Result:

MY336-a (30 μg/ml) blocked the positive chronotropic effect induced by isoproterenol hydrochloride at $10^{-9}$ g/ml by 84.9%.

**Claims**

1. A compound represented by the formula (I):

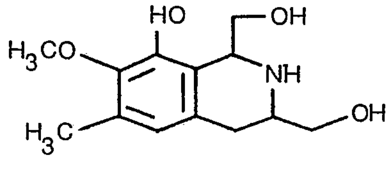

(I)

2. A process for producing a compound represented by the formula (I):

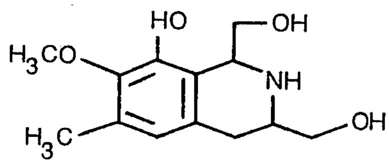

(I)

which comprises culturing for 1—12 days in a medium containing a carbon- and a nitrogen-source at a temperature of 25—40°, at a pH of around neutrality the microorganism *Streptomyces gabonae* ATCC 15282 capable of producing the compound of formula (I) thereby forming and accumulating the said compound in the culture liquor, and recovering the compound therefrom.

3. A pharmaceutical composition which comprises an effective amount of a compound represented by the formula (I)

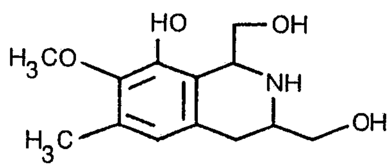

(I)

and at least one pharmaceutical carrier or diluent.

4. Use of compound represented by the formula (I)

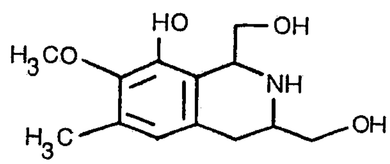

(I)

for the manufacture of a medicament for a therapeutic application which blocks a sympathetic nerve receptor.

**Patentansprüche**

1. Eine Verbindung der Formel (I):

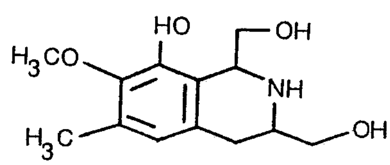

(I)

2. Ein Verfahren zur Herstellung einer Verbindung der Formel (I):

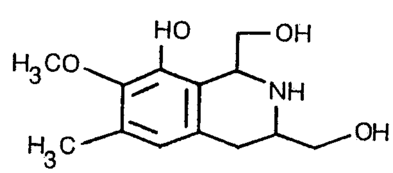

(I)

das ein- bis zwölftägiges Züchten des zur Erzeugung der Verbindung der Formel (I) defähigten Mikroorganismus Streptomyces gabonae ATCC 15282 in einem eine Kohlenstoff- und Stickstoffquelle enthaltenden Medium bei einer Temperatur von 25 bis 40°C und einem etwa neutralen pH-Wert, wobei sich besagte Verbindung in der Kulturbrühe bildet und anreichert, und Wiedergewinnung der Verbindung daraus umfaßt.

3. Ein Arzeneimittel, das eine wirksame Menge einer Verbindung der Formel (I):

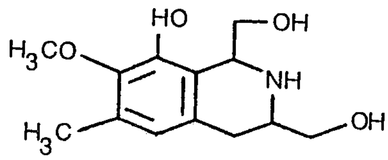 (I)

und mindestens einen pharmazeutischen Träger oder ein Verdünnungsmittel umfaßt.

4. Verwendung der Verbindung

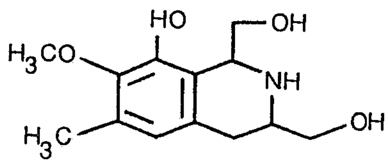 (I)

zur Herstellung eines Arzneimittels zur therapeutischen Anwendung, das einen Rezeptor des Sympathikus-Nerves blockiert.

**Revendications**

1. Composé représenté par la formule (I):

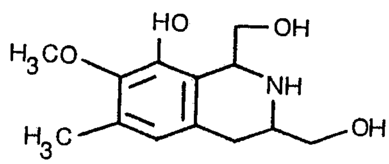 (I)

2. Procédé de préparation d'un composé représenté par la formule (I):

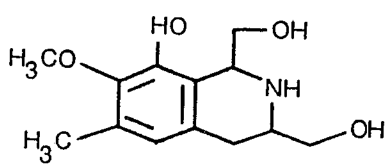 (I)

qui comprend la culture, pendant un à douze jours, dans un milieu contenant une source d'azote et une source de carbone, à une température de 25—40° et à un pH situé aux alentours de la neutralité, du micro-organisme *Streptomyces gabonae* ATCC 15282, capable de produire le composé de formule (I), la formation et l'accumulation dudit composé dans le milieu de culture, et la récupération du composé à partir de celui-ci.

3. Composition pharmaceutique qui comprend une quantité efficace d'un composé représenté par la formule (I):

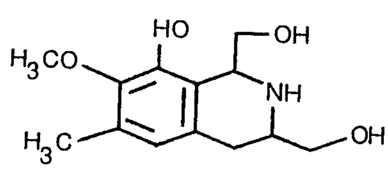 (I)

et au moins un véhicle ou diluant pharmaceutique.

4. Utilisation du composé:

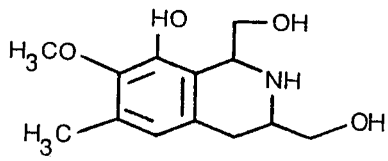

(I)

pour la fabrication d'un médicament, destiné à une application thérapeutique et qui bloque des récepteurs du système nerveux sympathique.

Fig. 1

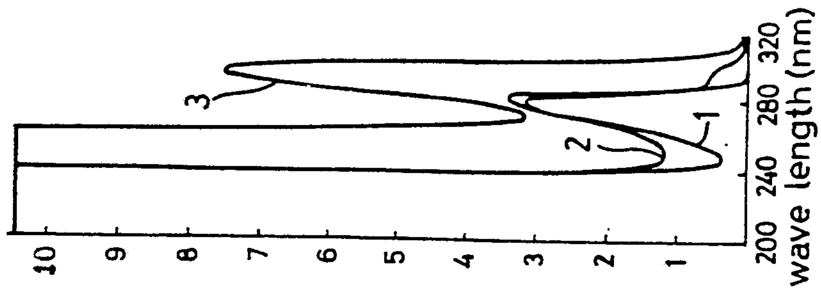
10
9
8
7
6
5
4
3
2
1
200
240
280
320
wave length (nm)
1
2
3

Fig. 2

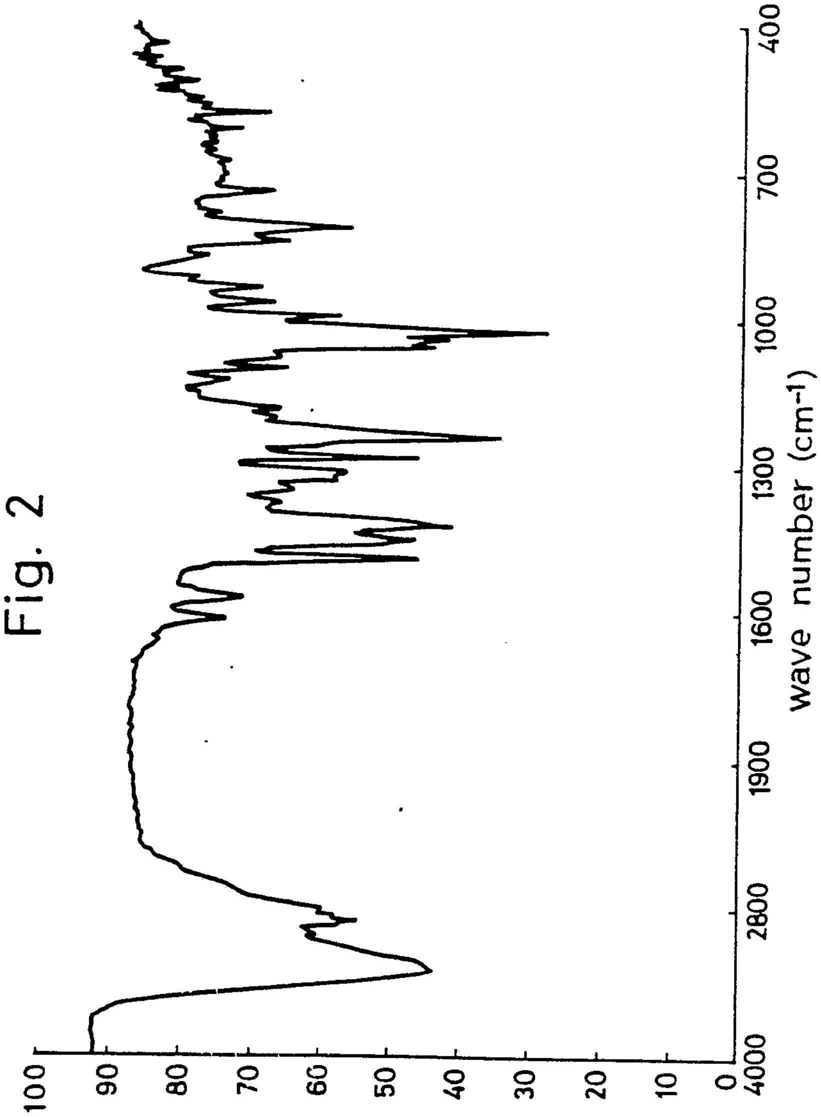
100
90
80
70
60
50
40
30
20
10
0
4000
2800
1900
1600
1300
1000
700
400
wave number (cm-1)